# EUROPÄISCHE PATENTSCHRIFT

(11) **EP 1 578 491 B1**
(45) Veröffentlichungstag und Bekanntmachung des Hinweises auf die Patenterteilung: **18.05.2016**
(21) Anmeldenummer: 03785861.0
(22) Anmeldetag: 17.12.2003
(51) Int. Cl.: A61N 1/37, A61N 1/362

(54) **IMPLANTIERBARER HERZSTIMULATOR**
IMPLANTABLE CARDIAC STIMULATOR
STIMULATEUR CARDIAQUE IMPLANTABLE

(30) Priorität: 20.12.2002 DE 10261821
(43) Veröffentlichungstag der Anmeldung: 28.09.2005
(73) Patentinhaber: BIOTRONIK SE & Co. KG, 12359 Berlin (DE)
(72) Erfinder: HANSEN, Sven, 14195 BERLIN (DE); HAUSER, Tino, 2583 NH The Hague (NL)
(74) Vertreter: Galander, Marcus
(86) Internationale Anmeldenummer: PCT/EP2003/014429
(87) Internationale Veröffentlichungsnummer: WO 2004/058350

(56) Entgegenhaltungen:
- US-A- 5 626 621
- US-A- 5 713 929
- US-B1- 6 185 459
- US-B1- 6 238 422

## Beschreibung

Die Erfindung betrifft einen implantierbaren Herzstimulator, insbesondere einen Herzschrittmacher oder Kardioverter/Defibrillator (ICD). Der Herzstimulator umfasst eine ventrikuläre Detektionseineit, die mit einer intrakardialen Elektrode zu verbinden und ausgebildet ist, ventrikuläre Ereignisse zu erfassen und zu detektieren. Außerdem umfasst der Herzstimulator eine ventrikuläre Stimulationseinheit, die mit einer ventrikulären Elektrode zu verbinden und ausgebildet ist, ventrikuläre Stimulationsimpulse für die Abgabe an den Ventrikel eines Herzens zu erzeugen. Weiterhin umfasst der Herzstimulator eine Steuereinheit, die mit der ventrikulären Detektionseinheit und mit der ventrikulären Stimulationseinheit verbunden und ausgebildet ist, die ventrikuläre Stimulationseinheit im VVI-Modus (ventrikel-inhibiert) derart anzusteuern, dass ein ventrikulärer Stimulationsimpuls zu einem durch eine Stimulationsrate vorgegebenen Zeitpunkt ausgelöst wird, sofern ein nicht durch detektieren einer natürlichen ventrikulären Kontraktion mittels der ventrikulären Detektionseinheit innerhalb eines vorgegebenen Zeitfensters inhibiert wird. Das vorgegebenen Zeitfenster wird häufig als Escape-Intervall bezeichnet.

Implantierbare Herzschrittmacher, die zur Stimulation eines menschlichen Herzens in einem ventrikel-inhibierten Modus ausgelegt sind, sind grundsätzlich bekannt. Die verschiedenen Stimulations- und Sensingmodi werden in der Regel einheitlich mit einem Drei-Buchstaben-Code bezeichnet, von denen der erste Buchstabe den Stimulationsort bezeichnet (V = Ventrikel, A = Atrium, D = Ventrikel und Atrium), der zweite Buchstabe den Ort des Sensing bezeichnet (V = Ventrikel, A = Atrium, D = Ventrikel und Atrium) und der dritte Buchstabe die Betriebsart (I = Inhibiert, T = Getriggert, D = sowohl Inhibiert als auch Getriggert). Insbesondere für ZweiKammer-Herzschrittmacher im DDD-Modus ist es auch bekannt, eine ventrikuläre Stimulation synchron zu einer möglichst natürlichen atrialen Herzrate vorzunehmen. Falls im Atrium keine gesunde, natürliche Herzrate wahrzunehmen ist, beispielsweise im Falle einer atrialen Tachykardie oder einer atrialen Fibrillation, weisen grundsätzlich atriumsynchrone Herzschrittmacher häufig ein Modeswitching von einer atriumsynchronen ventrikulären Stimulation zu einer atriumasynchronen Stimulation im VVI-Modus auf, falls eine wahrgenommenen atriale Rate außerhalb zulässiger Grenzen liegen.

Weiterhin ist es bekannt, ventrikuläre Tachykardien im Rahmen einer Kardioversionstherapie durch Stimulation mit einer Stimulationsrate, die über der Tachykardierate liegt, zu behandeln. Auf diese Weise lässt sich eine Reihe subraventrikulärer und ventrikulärer Tachykardien durchbrechen. Ziel ist es insbesondere Reentry-Kreise dadurch zu unterbrechen, indem ein ektopes Reizbildungszentrum vorzeitig durch Stimulation depolarisiert wird, bevor ein trinsischer Reiz wirksam werden kann. Die Stimulationsfrequenz für die Overdrive-Stimulation wird im Allgemeinen so gewählt, dass sie zehn bis fünfzig Impulse pro Minute höher liegt als die zu terminierende Tachykardie. Zusammengefasst sind aus dem Stand der Technik Ansätze bekannt, wie auf vorliegende Tachykardien zu reagieren ist. Eine Reaktion auf atriale Tachykardien ist beispielsweise das Mode-Switching in den VVI-Modus und als eine Reaktion auf eine ventrikuläre Tachykardie kann das Auslösen einer Overdrive-Stimulation als Kardioversionstherapie vorgesehen sein. Ein Gerät nach der Präambel von Anspruch 1 ist aus US5713929 bekannt.

Ziel der vorliegenden Erfindung ist es, einen Herzstimulator anzugeben, mit dem das Auftreten ventrikulärer Tachykardien möglichst von vorn herein unterbunden werden kann.

Erfindungsgemäß wie in Anspruch 1 definiert, wird diese Aufgabe durch einen implantierbaren Herzstimulator der Eingangs genannten Art gelöst, bei welchem die Steuereinheit ausgebildet ist, eine Stimulationsrate vorzugeben, die höher ist als eine einem physiologischen Bedarf angemessene, intrinsische Rate. Diese vorgegebene Stimulationsrate ist vorzugsweise variabel und wird im folgenden auch als Überstimulationsrate bezeichnet, um kenntlich zu machen, dass die Überstimulationsrate höher ist, als eine dem physiologischen Bedarf angepasste Rate.

Im Unterschied zu allen bekannten Herzschrittmachern soll der erfindungsgemäße Herzschrittmacher somit ständig in einer Art Overdrive-Modus operieren, und nicht nur beim Vorliegen einer Tachykardie. Üblicherweise wird mit Overdrive-Rate eine Rate bezeichnet, die oberhalb einer Bezugsrate liegt, nämlich bei bekannten Kardiovertern oberhalb einer Tachykardierate. Für den hier beanspruchten Schrittmacher ist die Bezugsrate für die beanspruchte, permanente Overdrive-Stimulation keine pathologische Rate, sondern eine natürliche intrinsische Rate, die zum Beispiel einer gesunden atrialen Herzrate entspricht oder auf an sich bekannte Weise aus den physiologischen Bedarf eines Patienten kennzeichnenden Parametern oder Messwerten abgeleitet ist. Die Begriffe Überstimulationsrate oder Overdrive-Stimulationsrate im hier verwendeten Sinne sollen nicht mit der bekannte Overdrive-Stimulationsrate verwechselt werden, die zur Tachykardiebehandlung eingesetzt wird. Overdrive-Stimulationsrate im herkömmlichen Sinn, meint eine Rate, die größer ist als eine Tachykardierate, während hier eine Rate gemeint ist, die nur wenig größer ist als eine gesunde, physiologisch adäquate Rate und jedenfalls viel kleiner, als eine Tachycardierate.

Der letzt bekannte Fall betrifft einen ratenadaptiven Schrittmacher, bei dem eine Stimulationsrate für den Herzschrittmacher so eingestellt wird, dass die Stimulationsrate vom physiologischen Bedarf eines Patienten abhängt, also beispielsweise mit zunehmender Anstrengung ansteigt, wie dies auch bei gesundem Herzen der Fall ist.

Gemäß dem Gedanken der dauerhaften Überstimulation ist die Steuereinheit des implantierbaren Herzschrittmachers in einer Ausführungsvariante dazu ausgebildet, eine feste Stimulationsrate zwischen 70 und 80 Stimulationsimpulsen oder Schlägen pro Minute vorzugeben, insbesondere eine Rate von etwa 80 pro Minute. Eine derartige Stimulationsrate liegt etwa zehn bis zwanzig Schläge pro Minute oberhalb einer natürlichen Herzrate eines Patienten im Ruhezustand.

In einer bevorzugten Ausführungsvariante ist die Steuereinheit des implantierbaren Herzschrittmachers dazu ausgebildet, die Überstimulationsrate (Overdrive-Stimulationsrate) für die dauerhafte Überstimulation automatisch zu bilden. Dazu ist es insbesondere vorgesehen, dass die Steuereinheit ausgebildet ist, die Überstimulationsrate in Abhängigkeit mittelbar oder unmittelbar erfasster Überleitungen atrialer Reize über eine AV-Knoten eines Herzens vom Atrium zum Ventrikel des Herzens derart vorzugeben, dass die Anzahl der Überleitungen oder die Anzahl der übergeleiteten Reize innerhalb vorgegebener Zeit oder bezogen auf eine vorgegebene Anzahl ventrikulärer Ereignisse ein vorgegebenes Maß nicht überschreitet. Die ventrikulären Ereignisse können sowohl stimulierte als auch natürliche Ereignisse sein. Die Steuereinheit ist somit so ausgebildet, dass sie durch Auswertung der Ereignisse oder anderer Messwerte in einem retrospektiven Zeitraum automatisch eine patientenindividuelle optimale Überstimulations-Grundrate ermittelt. Mit Überstimulations-Grundrate ist hiermit die im Ruhezustand des Patienten vorzusehende Überstimulationsrate gemeint. Diese kann im Rahmen einer Ratenadaption im Falle erhöhter körperlicher Anstrengung des Patienten erhöht werden.

Die Wahl des retrospektiven Zeitraums entsprechend der vorgenannten vorgegebenen Zeit oder der vorgegebenen Anzahl ventrikulärer Ereignisse kann vorzugsweise variabel vorgegeben werden.

Um eine möglichst schnelle Anpassung der Überstimulationsrate zu erzielen, ist es vorzugsweise vorgesehen, die Überstimulationsrate bereits dann zu erhöhen, wenn nur eine einzige Überleitung oder ein einziger übergeleiteter Reiz durch die Steuereinheit erfasst wird. Die Steuereinheit ist in diesem Falle so ausgebildet, dass sie die Überstimulationsrate automatisch erhöht, sobald eine Überleitung oder ein übergeleiteter Reiz erfasst ist.

Vorzugsweise erhöht die Steuereinheit die Überstimulationsrate nur für einen bestimmten Zeitraum und senkt sie danach wieder ab. Auf diese Weise wird vermieden, dass eine zu hohe Überstimulationsrate dauerhaft eingestellt wird. Auf der anderen Seite wird die Zahl möglicher Überleitungen wirksam reduziert. In einer alternativen bevorzugten Ausführungsform ist die Steuereinheit ausgebildet, die Stimulationsrate zu erhöhen, sobald zehn bis zwanzig Prozent Überleitungen oder zehn bis zwanzig Prozent übergeleitete Reize bezogen auf eine Gesamtzahl ventrikulärer Ereignisse im zurückliegenden Zeitraum erfasst sind. Mit anderen Worten stellt die Steuereinheit eine erhöhte Stimulationsrate ein, sobald die Gesamtzahl erfasster ventrikulärer Ereignisse in einem retrospektiven Zeitraum 10% bis 20% solcher Ereignisse zeigt, die auf Überleitungen beruhen.

Auch im Zusammenhang mit der zuletzt genannten, alternativ bevorzugten Ausführungsvariante ist es vorzugsweise vorgesehen, eine Erhöhung der Überstimulationsrate nach vorgegebener Zeit gegebenenfalls in Schritten wieder rückgängig zu machen.

In einer weiteren alternativen Ausführungsform ist die Steuereinheit dazu ausgebildet, die Überstimulationsrate in Abhängigkeit der Anzahl von Episoden ventrikulärer Tachykardie innerhalb eines vorgegebenen Zeitraums oder bezogen auf eine vorgegebene Anzahl ventrikulärer Ereignisse einzustellen. Ein Vorteil dieser Ausführungsvariante gegenüber alternativen Ausführungsvarianten besteht insbesondere darin, dass sich Tachykardieepisoden leicht detektieren lassen.

Vorzugsweise ist die Steuereinheit gemäß der letzt genannten Ausführungsvariante dazu ausgebildet, die Variable Stimulationsrate zu erhöhen, wenn die Anzahl von Episoden ventrikulärer Tachykardie einen vorgegebenen Grenzwert überschreitet. Dieser vorgegebene Grenzwert ist in einer besonders bevorzugten Ausführungsvariante 5 Prozent episodenventrikulärer Tachykardie bezogen auf die Gesamtzahl ventrikulärer Ereignisse in einem retrospektiven Beobachtungszeitraum.

Auch bei dieser letzt genannten Ausführungsvariante ist es vorzugsweise vorgesehen, die Überstimulationsrate nach einer Erhöhung wieder abzusenken. Wie in allen vorgenannten Fällen kann das allmähliche Absenken der erhöhten Überstimulationsrate nach vorgegebener Zeit in einem Schritt erfolgen oder in mehreren Schritten nach jeweils vorgegebener Zeit. Die Zeitvorgaben können dabei in Minuten oder Stunden angegeben sein oder aber auch als vorgegebene Anzahl von Herzzyklen.

In einer besonders bevorzugten Ausführungsvariante ist die Steuereinheit mit einem Sensor verbunden, mit dem ein von der körperlichen Aktivität eines Patienten abhängiger Messwert zu ermitteln ist. Die Steuereinheit ist in diesem Falle dazu ausgebildet, in Abhängigkeit des Messwertes eine an den physiologischen Bedarf angepasste Stimulationsrate zu ermitteln und eine entsprechende über dieser liegende Überstimulationsrate einzustellen.

Die Erfindung soll nun an Hand eines Ausführungsbeispiels mit Hilfe der Zeichnungen näher erläutert werden. In der Zeichnung zeigen die Figuren:
- Figur 1:: eine Übersichtsdarstellung eines Zweikammer-Herzschrittmachers mit angeschlossenen und implantierten atrialen und ventrikulären Elektroden;
- Figur 2:: eine grobschematische Blockdarstellung eines erfindungsgemäßen Herzschrittmachers.

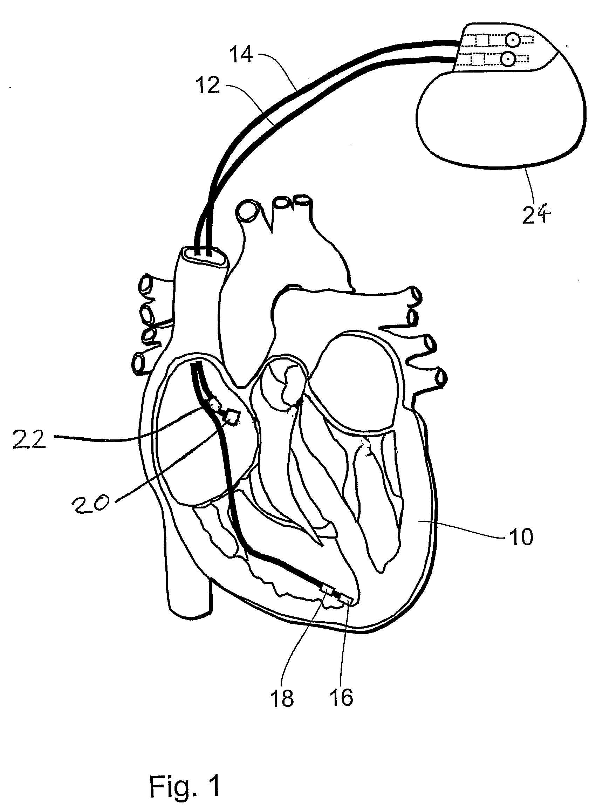

In Figur 1 ist schematisch ein menschliches Herz 10 dargestellt, in dessen Ventrikel eine ventrikuläre Elektrodenleitung 12 und in dessen Atrium eine atriale Elektrodenleitung 14 eingeführt sind. Die ventrikuläre Elektrodenleitung 12 besitzt eine ventrikuläre Spitzenelektrode 16 und eine ventrikuläre Ringelektrode 18. Die atriale Elektrodenleitung 14 besitzt eine atriale Spitzenelektrode 20 und eine atriale Ringelektrode 22. Die Elektrodenleitungen 12 und 14 sind an einen Zweikammer-Herzschrittmacher 24 angeschlossen.

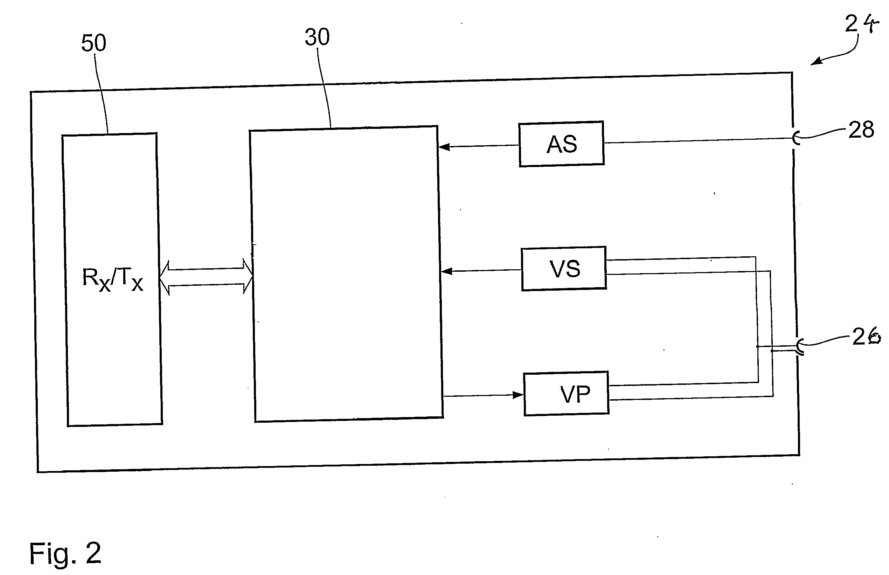

In Figur 2 sind die wesentlichen Komponenten des Herzschrittmachers 24 dargestellt. Dies sind ein bipolarer Elektrodenleitungsanschluss 26 für eine ventrikuläre Elektrodenleitung sowie ein uni- oder bipolare Elektrodenleitungsanschluss 28 für eine atriale Elektrodenleitung. In der dargestellten einfachen Ausführungsvariante des Herzschrittmachers 24 sind weiterhin eine ventrikuläre Detektionseinheit VS sowie eine ventrikuläre Stimulationseinheit VP vorgesehen, die über den Elektrodenleitungsanschluss 26 mit einer ventrikulären Elektrodenleitung mit der Elektrodenleitung 12 aus Figur 1 verbunden sind. Die ventrikuläre Detektionseinheit VS dient der Aufnahme elektrischer Signale des Herzens beispielsweise eines intrakardialen Elektrokardiogramms. Die ventrikuläre Stimulationseinheit VS umfasst einen Stimulationsimpulsgenerator mit zugehörigen Energiespeichern, Ladungspumpe usw, wie aus dem Stand der Technik bekannt ist und geeignet ist, um ventrikuläre Stimulationsimpulse zu generieren und erforderlichenfalls abzugeben. Zur Auslösung ventrikulärer Stimulationsimpulse ist die ventrikuläre Stimulationseinheit VS mit einer Steuereinheit 30 verbunden. Die Steuereinheit 30 ist außerdem mit der ventrikulären Detektionseinheit VS verbunden sowie mit einer atrialen Detektionseinheit AS. Die atriale Detektionseinheit AS ist über den Elektrodenleitungsanschluss 28 mit einer atrialen Elektrodenleitung wie der Elektrodenleitung 14 zu verbinden.
Zur Kommunikation mit externen Geräten ist die Steuereinheit 30 mit einer Telemetrieeinheit Rx/Tx für die drahtlose Kommunikation beispielsweise intrakardial aufgenommener Elektrokardiogramme aus dem Herzschrittmacher 24 an ein externes Gerät oder zur Übertragung von Programmierbefehlen von einem externen Gerät zu dem Herzschrittmacher 24.

Nicht dargestellt ist eine potentiell vorzusehende atriale Stimulationseinheit, die den Herzschrittmacher 24 in die Lage versetzt, auch das Atrium eines Herzens zu stimulieren. Ein derartiger Zweikammer-Herzschrittmacher im engeren Sinne kann beispielsweise in dem an sich bekannten DDD-Modus betrieben werden. Nicht weiter dargestellte Mittel zur Erfassung der intrakardialen Impedanz können bei einem solchen Schrittmacher dazu verwendet werden, zum einen unmittelbar nach Abgabe eines Stimulationsimpulses einen Stimulationserfolg zu detektieren (Capture Detection) um bei möglicherweise ausbleibendem Stimulationserfolgt sofort einen Back-Up-Impuls auslösen zu können. Weiterhin wird im vorliegenden Fall nicht näher auf die an sich bekannten Mittel für das Ermitteln des physiologischen Bedarfs eines Patienten im Fall körperlicher Anstrengung eingegangen. Solche Mittel können beispielsweise ein Blutsauerstoffsensor sein oder wiederum ein Impedanzsensor, der aus dem Integral eines intrakardialen Impedanzverlaufs ein Steuersignal für die Herzrate ableitet, so dass die Herzrate im Sinne eines geschlossenen Regelkreises (Close Loop Stimulation) an den physiologischen Bedarf angepasst werden kann.

Im vorliegenden Fall interessiert insbesondere ein VVI-Modus, in dem der Herzschrittmacher 24 betrieben werden kann. Die für diese Betriebsart zu Grunde zu legende Stimulationsrate kann im Sinne eines ratenadaptiven Herzschrittmachers vom physiologischen Bedarf abhängig gesteuert werden oder eine fest vorgegebene Grundrate sein. Bevorzugt ist jedoch ein ratenadaptiver Herzschrittmacher 24. Diese hierzu erforderliche Bestimmung des physiologischen Bedarfs kann beispielsweise wie angedeutet mit Hilfe eines Blutsauerstoffssensors erfolgen, oder durch Auswertung eines mit Hilfe entsprechender Sensoren aufgenommenen intrakardialen Impedanzverlaufes. Die Sensoren können dabei die Elektroden 16 bis 22 einschließlich des Gehäuses des Herzschrittmachers 24 sein. Die Auswertung des intrakardialen Impedanzverlaufs geschieht in der Steuereinheit 30.

Des weiteren kann je nach Patient eine gesunde intrinsische Herzrate im Atrium des Herzens 10 mit Hilfe der atrialen Detektionseinheit AS erfasst werden.

Für den hier interessierenden VVI-Betrieb des Herzschrittmachers 24 wird zunächst eine Basisrate auf Grundlage beispielsweise einer mit der atrialen Detektionseinheit AS erfassten atrialen Herzrate oder durch Berechnung auf Grundlage des physiologischen Bedarfs des Patienten ermittelt. Die Steuereinheit 30 ist dann so ausgebildet, aus der variablen Basisrate eine variable Stimulationsrate als Überstimulationsrate zu bilden, die beispielsweise 10 bis 20 Schläge pro Minute höher ist, als die dem physiologischen Bedarf angepasste Basisrate. Die Steuereinheit 30 ermittelt die genaue Größe der Überstimulationsrate anhand erfasster Reizüberleitungen vom Atrium zum Ventrikel. Solche Reizüberleitungen lassen sich beispielsweise durch Auswertung der im Atrium mittels der atrialen Detektionseinheit AS aufgenommenen Signale im Vergleich zu entsprechenden im Ventrikel mittels der ventrikulären Detektionseinheit VS erfassten Signalen ermitteln. Die durch die Steuereinheit 30 zu ermittelnde Überstimulationsrate wird erhöht, wenn in einem vorgegebenen retrospektiven Zeitraum (beispielsweise 20 Herzzyklen) mehr als 10% oder 20% Reizüberleitungen vom Atrium zum Ventrikel detektiert werden. Falls umgekehrt in diesem retrospektiven Zeitraum keine Reizüberleitungen detektiert werden, wird die Überstimulationsrate allmählich abgesenkt. Durch Absenken der Überstimulationsrate erhöht sich die Wahrscheinlichkeit der Überleitung atrialer Reize zum Ventrikel. Sobald eine vorgegebene Anzahl solcher Überleitungen in dem vorgegebenen retrospektiven Zeitraum erfasst wird, wird die Überstimulationsrate wieder erhöht. Auf diese Weise werden natürlich Überleitungen von Reizen vom Atrium zum Ventrikel weit es gehend unterbunden und damit auch soweit wie möglich das Auftreten ventrikulärer Tachykardien.

## Patentansprüche

1. Implantierbarer Herzstimulator, insbesondere Herzschrittmacher oder Kardioverter/Defibrillator (ICD), mit einer ventrikulären Detektionseinheit (VS), die mit einer intrakardialen Elektrode (16; 18) zu verbinden und ausgebildet ist, ventrikuläre Ereignisse zu erfassen und zu detektieren, sowie mit einer ventrikulären Stimulationseinheit (VP), die mit einer ventrikulären Elektrode (16; 18) zu verbinden und ausgebildet ist, ventrikuläre Stimulationsimpulse für die Abgabe an den Ventrikel eines Herzens (10) zu erzeugen, und mit einer Steuereinheit (30), die mit der ventrikulären Detektionseinheit (VS) und mit der ventrikulären Stimulationseinheit (VP) verbunden und ausgebildet ist, die ventrikuläre Stimulationseinheit (VP) in einem VVI-Modus ventrikel-inhibiert derart anzusteuern, dass ein ventrikulärer Stimulationsimpuls zu einem durch eine Stimulationsrate vorgegebenen Zeitpunkt ausgelöst wird, sofern er nicht durch Detektieren einer natürlichen ventrikulären Kontraktion mittels der ventrikulären Detektionseinheit (VS) innerhalb eines vorgegebenen Zeitfensters inhibiert wird, wobei die Steuereinheit (30) ausgebildet ist, eine Stimulationsrate vorzugeben, die höher ist, als eine einem physiologischen Bedarf angemessene insbesondere intrinsische Rate, **dadurch gekennzeichnet dass** die Steuereinheit (30) ausgebildet ist, eine variable Stimulationsrate in Abhängigkeit mittelbar oder unmittelbar erfasster Überleitungen atrialer Reize über einen AV-Knoten eines Herzens vom Atrium zum Ventrikel des Herzens derart vorzugeben, dass die Anzahl der Überleitungen oder die Anzahl der übergeleiteten Reize innerhalb vorgegebener Zeit oder bezogen auf eine vorgegebene Anzahl ventrikulärer Ereignisse ein vorgegebenes Maß nicht überschreitet.

2. Herzstimulator nach Anspruch 1, **dadurch gekennzeichnet, dass** die Steuereinheit (30) ausgebildet ist, eine feste Stimulationsrate zwischen 70 und 90 pro Minute, vorzugsweise etwa 80 pro Minute vorzugeben.

3. Herzstimulator nach Anspruch 1, **dadurch gekennzeichnet, dass** die Steuereinheit (30) ausgebildet ist, die variable Stimulationsrate zu erhöhen, wenn die Anzahl der Überleitungen oder die Anzahl der übergeleiteten Reize das vorgegebene Maß überschreitet.

4. Herzstimulator nach Anspruch 1 oder 3, **dadurch gekennzeichnet, dass** das vorgegebene Maß eine einzige Überleitung oder ein einziger übergeleiteter Reiz ist.

5. Herzstimulator nach Anspruch 1 oder 3, **dadurch gekennzeichnet, dass** das vorgegebene Maß 10 bis 20% Überleitungen oder 10 bis 20% übergeleitete Reize bezogen auf eine Gesamtzahl ventrikulärer Ereignisse ist.

6. Herzstimulator nach einem der Ansprüche 1 bis 5, **dadurch gekennzeichnet, dass** die Steuereinheit (30) ausgebildet ist, eine variable Stimulationsrate in Abhängigkeit der Anzahl von Episoden ventrikulärer Tachykardie innerhalb eines vorgegeben Zeitraums oder bezogen auf eine vorgegebene Anzahl ventrikulärer Ereignisse einzustellen.

7. Herzstimulator nach Anspruch 6, **dadurch gekennzeichnet, dass** die Steuereinheit (30) ausgebildet ist, die variable Stimulationsrate zu erhöhen, wenn die Anzahl von Episoden ventrikulärer Tachykardie einen vorgegebenen Grenzwert überschreitet.

8. Herzstimulator nach Anspruch 7, **dadurch gekennzeichnet, dass** der vorgegebene Grenzwert 5% Episoden ventrikulärer Tachykardie bezogen auf die Gesamtzahl ventrikulärer Ereignisse ist.

9. Herzstimulator nach Anspruch 3, oder 7, **dadurch gekennzeichnet, dass** die Steuereinheit (30) ausgebildet ist, die variable Stimulationsrate solange allmählich abzusenken, bis wieder eine Erhöhung der variablen Stimulationsrate eintritt.

## Claims

1. An implantable cardiac stimulator, in particular a cardiac pacemaker or cardioverter/defibrillator (ICD), comprising a ventricular detection unit (VS) which is to be connected to an intracardiac electrode (16; 18) and is configured to record and detect ventricular events, and also comprising a ventricular stimulation unit (VP) which is to be connected to a ventricular electrode (16; 18) and is configured to produce ventricular stimulation pulses for delivery to the ventricle of a heart (10), and comprising a control unit (30) which is connected to the ventricular detection unit (VS) and to the ventricular stimulation unit (VP) and is configured to actuate the ventricular stimulation unit (VP) in a VVI mode in ventricle-inhibited fashion in such a way that a ventricular stimulation pulse is triggered at a moment in time predetermined by a stimulation rate if it is not inhibited by detection of a natural ventricular contraction by means of the ventricular detection unit (VS) within a predetermined time window, wherein the control unit (30) is configured to predetermine a stimulation rate which is higher than an in particular intrinsic rate appropriate to a physiological demand, **characterised in that** the control unit (30) is configured to predetermine a variable stimulation rate depending on indirectly or directly detected transconductions of atrial stimuli by way of an AV node of a heart from the atrium to the ventricle of the heart, in such a way that the number of transconductions or the number of transconducted stimuli within a predetermined time or in relation to a predetermined number of ventricular events does not exceed a predetermined degree.

2. The cardiac stimulator according to Claim 1, **characterised in that** the control unit (30) is configured to predetermine a fixed stimulation rate between 70 and 90 per minute, preferably about 80 per minute.

3. The cardiac stimulator according to Claim 1, **characterised in that** the control unit (30) is configured to increase the variable stimulation rate if the number of transconductions or the number of transconducted stimuli exceeds the predetermined degree.

4. The cardiac stimulator according to Claim 1 or 3, **characterised in that** the predetermined degree is a single transconduction or a single transconducted stimulus.

5. The cardiac stimulator according to Claim 1 or 3, **characterised in that** the predetermined degree is between 10 and 20% of transconductions or between 10 and 20% of transconducted stimuli in relation to a total number of ventricular events.

6. The cardiac stimulator according to one of Clams 1 to 5, **characterised in that** the control unit (30) is configured to set a variable stimulation rate depending on the number of episodes of ventricular tachycardia within a predetermined period of time or in relation to a predetermined number of ventricular events.

7. The cardiac stimulator according to Claim 6, **characterised in that** the control unit (30) is configured to increase the variable stimulation rate when the number of episodes of ventricular tachycardia exceeds a predetermined limit value.

8. The cardiac stimulator according to Claim 7, **characterised in that** the predetermined limit value is 5% of episodes of ventricular tachycardia in relation to the total number of ventricular events.

9. The cardiac stimulator according to Claim 3 or 7, **characterised in that** the control unit (30) is configured to gradually reduce the variable stimulation rate until an increase in the variable stimulation rate occurs again.

## Revendications

1. Stimulateur cardiaque implantable, notamment stimulateur cardiaque ou défibrillateur/cardioverteur (ICD), avec une unité de détection ventriculaire (VS) qui est à relier avec une électrode intracardiaque (16 ; 18) et est conçue pour saisir et détecter des événements ventriculaires, ainsi qu'avec une unité de stimulation ventriculaire (VP) qui est à relier avec une électrode ventriculaire (16 ; 18) et est conçue pour générer des impulsions de stimulation ventriculaires pour l'attribution à un ventricule du coeur (10), et avec une unité de commande (30) qui est reliée avec l'unité de détection ventriculaire (VS) et avec l'unité de stimulation ventriculaire (VP) et est conçue pour commander l'unité de stimulation ventriculaire (VP) dans un mode VVI de ventricule inhibé de telle sorte qu'une impulsion de stimulation ventriculaire est libéré à un instant prédéfini par un taux de stimulation, dans la mesure où elle n'est pas inhibée par une détection d'une contraction ventriculaire naturelle au moyen de l'unité de détection ventriculaire (VS) pendant un laps de temps prédéfini, où l'unité de commande (30) est conçue pour prédéfinir un taux de stimulation qui est supérieur à un taux approprié, notamment intrinsèque, à un besoin physiologique, **caractérisé en ce que** l'unité de commande (30) est conçue de sorte à prédéfinir un taux de stimulation variable en fonction de détections directes ou indirectes de transferts de stimuli atrials par l'intermédiaire de noeuds AV du coeur de l'atrium vers le ventricule du coeur de telle manière que le nombre de transferts ou le nombre des stimuli transférés pendant un temps prédéfini ou par rapport à un nombre prédéfini d'événements ventriculaires ne dépassent pas une mesure prédéfinie.

2. Stimulateur cardiaque selon la revendication 1, **caractérisé en ce que** l'unité de commande (30) est conçue pour prédéfinir un taux de stimulation fixe entre 70 et 90 par minute, de préférence environ 80 par minute.

3. Stimulateur cardiaque selon la revendication 1, **caractérisé en ce que** l'unité de commande (30) est conçue pour élever le taux de stimulation variable lorsque le nombre des transferts ou le nombre des stimuli transférés dépasse la mesure prédéfinie.

4. Stimulateur cardiaque selon les revendications 1 ou 3, **caractérisé en ce que** la mesure prédéfinie est un seul transfert ou un seul stimulus transféré.

5. Stimulateur cardiaque selon les revendications 1 ou 3, **caractérisé en ce que** la mesure prédéfinie est de 10 % à 20 % de transferts ou de 10 à 20 % de stimuli transférés par rapport au nombre total des événements ventriculaires.

6. Stimulateur cardiaque selon l'une des revendications 1 à 5, **caractérisé en ce que** l'unité de commande (30) est conçue de sorte à régler un taux de stimulation variable en fonction du nombre d'épisodes de tachycardie ventriculaire pendant un laps de temps prédéfini ou par rapport à un nombre prédéfini d'événements ventriculaires.

7. Stimulateur cardiaque selon la revendication 6, **caractérisé en ce que** l'unité de commande (30) est conçue pour élever le taux de stimulation variable lorsque le nombre d'épisodes de tachycardie ventriculaires dépasse une valeur limite prédéfinie.

8. Stimulateur cardiaque selon la revendication 7, **caractérisé en ce que** la valeur limite prédéfinie est 5 % d'épisodes de tachycardie ventriculaire par rapport au nombre total d'événements ventriculaires.

9. Stimulateur cardiaque selon la revendication 3, ou 7, **caractérisé en ce que** l'unité de commande (30) est conçue pour diminuer progressivement le taux de stimulation variable jusqu'à ce que de nouveau une augmentation du taux de stimulation variable se produise.
